# EUROPÄISCHE PATENTSCHRIFT

(11) **EP 2 308 401 B1**
(45) Veröffentlichungstag und Bekanntmachung des Hinweises auf die Patenterteilung: **18.04.2018**
(21) Anmeldenummer: 10013490.7
(22) Anmeldetag: 11.10.2010
(51) Int. Cl.: A61B 17/42

(54) **Uterus-Manipulator**
Uterus manipulator
Manipulateur d'utérus

(30) Priorität: 12.10.2009 DE 102009049169
(43) Veröffentlichungstag der Anmeldung: 13.04.2011
(73) Patentinhaber: Karl Storz SE & Co. KG, 78532 Tuttlingen (DE)
(72) Erfinder: Hahn, Martin, 88605 Boll (DE); Doll, Frank, 78607 Talheim (DE); Graf, Anne-Kathrin, 78570 Mühlheim-Stetten (DE); Steckeler, Sven, 72108 Rottenburg (DE)

(56) Entgegenhaltungen:
- WO-A1-93/18713
- DE-A1-102005 042 338
- US-A- 5 578 048
- US-A- 5 730 725
- US-A1- 2008 109 010
- US-B1- 6 234 958

## Beschreibung

Die vorliegende Erfindung bezieht sich auf ein mikroinvasives medizinisches Instrument, insbesondere auf einen Uterus-Manipulator.

In der US 5,104,377 ist eine Vorrichtung zum Einführen von Fluiden oder anderen Instrumenten in den Uterus beschrieben. Die Vorrichtung umfasst zwei Ballone zur Anordnung innerhalb bzw. außerhalb der cervix uteri.

In der US 5,643,311 ist ein Uterus-Manipulator beschrieben, dessen distaler Abschnitt über ein Gelenk bewegbar ist. Das distale Ende weist eine weiche Siliconspitze auf.

Herkömmliche Uterus-Manipulatoren und andere Vorrichtungen zur Einführung in den Uterus oder andere Körperhöhlen weisen dünne Enden auf, die auch bei Ausführung in Silicon oder einem anderen weichen Material eine punktuelle Belastung der empfindlichen Schleimhäute des Uterus oder anderer Organe ermöglichen und damit ein Verletzungsrisiko darstellen. Sie erweisen sich in der Reinigung und Wartung als aufwändig.

Die US 5,578,048 zeigt einen chirurgische Vorrichtung für Körpergewebe, die einen Endballon aufweist, der über einen Kanal aufblasbar ist. Zusätzlich zeigt dieses Patent auch ein Gelenk proximal des Ballons.

Die US 5730725 beschreibt ein endoskopisches Multifunktionsinstrument, das wiederum zwei Ballons aufweist, und zusätzlich auch einen Kanal, um ein Fluid von proximal nach distal zu leiten.

In der US 2008/0109010 A1 wie auch US 5,730,725 ist ein Uterus-Manipulator offenbart, der einen Endballon am distalen Ende seines Schafts und einen Stützballon beabstandet vom distalen Ende des Schafts aufweist, sowie einen Kanal zum Aufblasen mindestens des Stützballons.

Die DE 10 2005 042 338 A1zeigt eine Kathetereinrichtung, die wiederum zwei Ballons aufweist, zusätzlich einen Kanal zur Befüllung des oder der Ballons. Ferner offenbart DE 10 2005 042 338 A1 zwischen den Ballons eine Öffnung, um einen Nebenkatheter oder ein Spülmedium in das Operationsgebiet einzudringen.

Eine Aufgabe der vorliegenden Erfindung besteht darin, ein verbessertes mikroinvasives medizinisches Instrument, insbesondere einen verbesserten Uterus-Manipulator und einen verbesserten distalen Abschnitt für ein solches mikroinvasives medizinisches Instrument zu schaffen.

Diese Aufgabe wird durch die Gegenstände der unabhängigen Ansprüche gelöst.

Weiterbildungen sind in den abhängigen Ansprüchen angegeben.

Verschiedene Ausführungsformen der vorliegenden Erfindung beruhen auf der Idee, an einem distalen Ende eines Schafts eines mikroinvasiven medizinischen Instruments oder eines distalen Abschnitts für ein mikroinvasives medizinisches Instrument einen Endballon vorzusehen, der das distale Ende des Instruments bzw. des distalen Abschnitts für das Instrument vollständig umschließt. Der Endballon umschließt das distale Ende des Schafts in dem Sinne vollständig, dass er den Schaft nicht nur azimutal bezogen auf die Längsachse des Schafts vollständig umgibt sondern auch die gesamte distale Stirnfläche des Instruments bildet. Der Endballon weist proximal jedenfalls eine Öffnung auf, in der der Schaft des Instruments angeordnet ist, so dass das distale Ende des Schafts innerhalb des Endballons und proximale Abschnitte des Schafts außerhalb des Endballons angeordnet sind.

Der Endballon ist aufblasbar bzw. durch Einleiten eines Fluids expandierbar und ist damit besonders atraumatisch. Selbst wenn der Endballon im aufgeblasenen bzw. expandierten bzw. mit einem Fluid gefüllten Zustand ein Organ von innen oder außen berührt und auf dieses eine Kraft ausübt, wird diese Kraft durch die großflächige und elastische Haut bzw. Wand des Ballons auf eine so große Fläche verteilt, dass nur ein minimaler Druck entsteht. Aufgrund der Geschmeidigkeit des Ballons bzw. seiner Wand werden auch lokale Druckspitzen wirkungsvoll vermieden. Dadurch sinkt das Verletzungsrisiko erheblich, insbesondere wenn beispielsweise wie im Fall eines Uterus-Manipulators eine Kraft ausgeübt werden soll und muss, um ein Organ zu bewegen.

Ein mikroinvasives medizinisches Instrument, insbesondere ein Uterus-Manipulator, umfasst einen Schaft und einen aufblasbaren Endballon an einem distalen Ende des Schafts, wobei der aufblasbare Endballon das distale Ende des Instruments vollständig umschließt. Das Instrument kann ferner einen Stützballon am Schaft aufweisen, der vom distalen Ende des Schafts beabstandet ist. Im Fall eines Uterus-Manipulators ist der Stützballon insbesondere ausgebildet, um im Gebährmutterhals (Cervix uteri) oder am oder beim inneren Muttermund (Ostium uteri internum) angeordnet zu werden. Zwischen dem Endballon und dem Stützballon kann eine seitliche Öffnung am Schaft vorgesehen sein, durch die ein Fluid (beispielsweise zur Pertubation) oder ein Gegenstand (beispielsweise ein endoskopisches Instrument) aus dem Schaft nach außen geschoben werden kann.

Im oder am Schaft können ein erster Kanal, dessen Lumen am distalen Ende mit dem Inneren des Endballons verbunden ist, und ein zweiter Kanal, dessen Lumen am distalen Ende mit dem Inneren des Stützballons verbunden ist, vorgesehen sein. Über diese beiden Kanäle können der Endballon und der Stützballon unabhängig voneinander aufgeblasen bzw. expandiert bzw. ihre Größen eingestellt werden. Alternativ ist ein einziger Kanal vorgesehen, der mit den Innenräumen sowohl des Endballons als auch des Stützballons verbunden ist, um beide Ballone mit einem Fluid füllen zu können.

Alternativ zur Aufblasbarkeit kann der Endballon mittels eines oder mehrerer Bänder, oder anderer Zugelemente und/oder anderer mechanischer Einrichtungen aufgespreizt, expandiert, entfaltet oder vergrößert werden. Gleiches gilt gegebenenfalls für den Stützballon. Gegebenenfalls können beide Ballone aufblasbar, beide mittels eines oder mehrerer Bänder oder anderer Zugelemente und/oder anderer mechanischer Einrichtungen oder je einer der beiden Ballone auf die eine Weise und einer auf die andere Weise expandiert, aufgespreizt, entfaltet oder vergrößert werden.

Beispielsweise weist der Endballon und/oder gegebenenfalls der Stützballon eine Membran aus einem elastischen Material auf, die im entspannten bzw. spannungsfreien oder spannungsarmen Zustand eine expandierte Form einnimmt. Ein oder mehrere Bänder oder andere Zugelemente sind so an der Membran befestigt, dass das Volumen und der Querschnitt des betreffenden Ballons durch Zug an dem oder den Bändern oder anderen Zugelementen vermindert werden können. Dazu sind das oder die Bänder oder andere Zugelemente beispielsweise an der Innenseite der Membran an einem oder mehreren Orten befestigt. Diese Orte liegen in der expandierten Form der Membran beispielsweise möglichst weit beabstandet von einer Längsachse des Instruments, wobei das oder die Bänder oder andere Zugelemente angeordnet sind, um einen Zug nach radial innen auszuüben. Alternativ sind das oder die Bänder oder andere Zugelemente beispielsweise an einem proximalen Ende oder Rand des Ballons bzw. der Membran befestigt und angeordnet und ausgerichtet, um einen Zug in Längsrichtung des Instruments nach proximal auszuüben.

Alternativ weist der Endballon und/oder gegebenenfalls der Stützballon eine Membran aus einem elastischen Material auf, die im entspannten bzw. spannungsarmen oder -freien Zustand ein kleines Volumen und einen kleinen Querschnitt aufweist. Das oder die Bänder oder andere Zugelemente sind so angeordnet und an der Membran befestigt, dass die Membran bei Zug daran in Längsrichtung des Instruments gestaucht wird, wobei die Membran so ausgebildet ist, dass sie dabei gleichzeitig radial expandiert wird. Dazu sind das oder die Bänder oder andere Zugelemente beispielsweise an einem proximalen Ende bzw. Rand des Ballon befestigt und angeordnet, um einen Zug in distaler Richtung auszuüben. Eine ähnliche Wirkung ist erzielbar mit einem Schubelement, das an der gleichen Stelle angreift und angeordnet und ausgebildet ist, um einen Schub in distaler Richtung ausüben.

Das Instrument kann ein Gelenk zwischen einem proximalen Abschnitt und einem distalen Abschnitt des Schafts aufweisen, wobei der Endballon und gegebenenfalls der Stützballon am distalen Abschnitt des Schafts angeordnet sind. Damit kann beispielsweise nach Einführen des distalen Abschnitts in Cervix und Uterus der Uterus bewegt werden. Dies ist unter anderem bei laparoskopischen Eingriffen im Beckenraum und im unteren Bauchraum oft erforderlich oder zumindest vorteilhaft. Durch den Endballon, der das distale Ende des Schafts vollständig umschließt, wird eine vom Uterus-Manipulator auf den Uterus ausgeübte Kraft großflächig verteilt. Dies minimiert das Verletzungsrisiko.

Um das Verletzungsrisiko insbesondere schon beim Einführen des Instruments weiter zu verringern, betragen alle Krümmungsradien am vom Endballon umschlossenen distalen Ende des Schafts mindestens ein Viertel, noch besser mindestens ein Drittel des Durchmessers des Schafts bzw. der maximalen Abmessung des Querschnitts des Schafts. Besonders vorteilhaft ist ein halbkugelförmiges Ende des Schafts, wobei die Krümmungsradien dem Durchmesser des insbesondere kreiszylindrischen Schafts entsprechen.

Zwischen einem proximalen Abschnitt des Schafts und einem distalen Abschnitt des Schafts kann eine Kupplung zur mehrmaligen lösbaren mechanischen und funktionalen Kopplung der beiden Abschnitte ausgebildet sein. Dabei ist insbesondere der proximale Abschnitt zur mehrmaligen Verwendung vorgesehen und ausgebildet (d.h. insbesondere autoklavierbar), während der distale Abschnitt zur einmaligen Verwendung bzw. zum Einmalgebrauch vorgesehen und ausgebildet ist. Bei Ausbildung des distalen Abschnitts zur einmaligen Verwendung ist dieser kostengünstig hergestellt, aufgrund seiner Materialien und Oberflächen nicht oder nicht wiederholt autoklavierbar und weniger robust als bei Ausbildung zur mehrmaligen Verwendung.

Ein distaler Abschnitt für ein mikroinvasives medizinisches Instrument, insbesondere für einen Uterus-Manipulator, umfasst einen Schaftabschnitt, eine Kupplung zum Koppeln des distalen Abschnitts mit einem proximalen Schaftabschnitt und einen aufblasbaren Endballon an einem distalen Ende des Schaftabschnitts, wobei der aufblasbare Endballon das distale Ende des Schaftabschnitts vollständig umschließt. Der distale Abschnitt kann ferner einen Stützballon am Schaftabschnitt aufweisen, der vom distalen Ende des Schaftabschnitts beabstandet ist.

Ferner kann der distale Abschnitt eine seitliche Öffnung am Schaftabschnitt zwischen dem Endballon und dem Stützballon aufweisen, durch die ein Fluid (beispielsweise zur Pertubation) oder ein Gegenstand (beispielsweise ein endoskopisches Instrument) aus dem Schaftabschnitt nach außen geschoben werden kann. Im oder am Schaftabschnitt können ein erster Kanal, dessen Lumen am distalen Ende mit dem Inneren des Endballons verbunden ist, und ein zweiter Kanal, dessen Lumen am distalen Ende mit dem Inneren des Stützballons verbunden ist, aufweisen.

Alle Krümmungsradien am distalen Ende des Schaftabschnitts betragen insbesondere mindestens ein Viertel oder mindestens ein Drittel des Durchmessers des Schaftabschnitts. Der distale Abschnitt ist insbesondere zur einmaligen Verwendung bzw. zum Einmalgebrauch und zur anschließenden Entsorgung ausgebildet.

### Kurzbeschreibung der Figuren

Nachfolgend werden Ausführungsformen anhand der beigefügten Figuren näher erläutert. Es zeigen:
- Figur 1: eine schematische Darstellung eines mikroinvasiven medizinischen Instruments, insbesondere eines Uterus-Manipulators;
- Figur 2: eine schematische Darstellung eines distalen Abschnitts für ein mikroinvasives medizinisches Instrument;
- Figur 3: eine weitere schematische Darstellung des distalen Abschnitts aus Figur 2.

### Beschreibung der Ausführungsformen

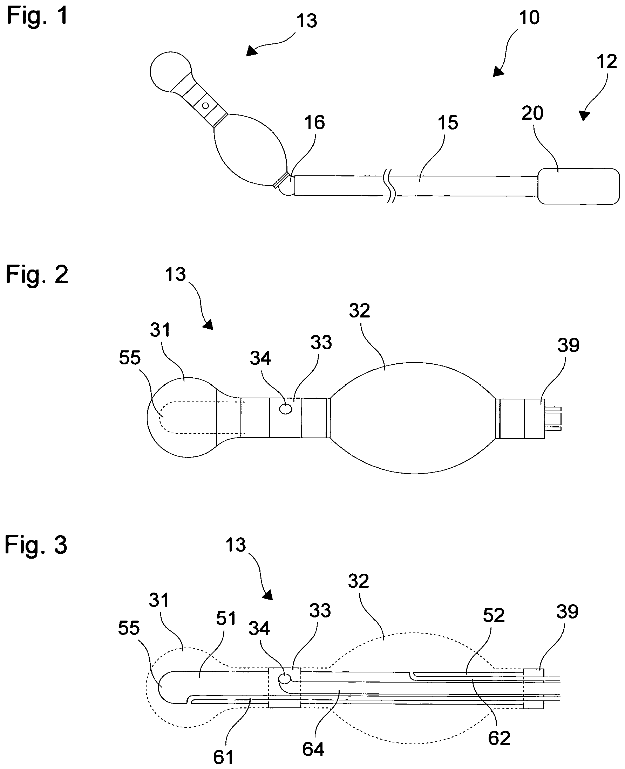

Figur 1 zeigt eine schematische Darstellung eines Uterus-Manipulators 10 als Beispiel eines mikroinvasiven medizinischen Instruments. Der Uterus-Manipulator 10 umfasst einen proximalen Abschnitt 12 und einen distalen Abschnitt 13 zur Einführung in Cervix und Uterus. Der Uterus-Manipulator 10 umfasst einen Schaft 15 der sich vom proximalen Abschnitt 12 bis zum distalen Abschnitt 13 erstreckt. Zwischen einem proximalen Abschnitt und einem distalen Abschnitt des Schafts 15 ist ein Gelenk 16 angeordnet.

Am proximalen Ende ist der Schaft 15 mit einer Griff-Einrichtung 20 verbunden, die in Figur 1 nur schematisch dargestellt ist. Die Griff-Einrichtung ist zur Handhabung, Führung und Steuerung des Uterus-Manipulators 10 ausgebildet und kann von der schematischen Darstellung in Figur 1 deutlich abweichende Merkmale aufweisen. Der distale Abschnitt 13 des Uterus-Manipulators 10 wird nachfolgend mit Bezug auf die Figuren 2 und 3 näher beschrieben. Das optionale Gelenk 16 ist für eine Anteversion und/oder Retroversion des Uterus um einen mittels der Griff-Einrichtung 20 steuerbaren Winkel ausgebildet.

Die Figuren 2 und 3 zeigen schematische Darstellung eines distalen Abschnitts 13 eines Uterus-Manipulators, wie er beispielsweise oben anhand der Figur 1 dargestellt wurde. In Figur 2 ist eine Seitenansicht des Uterus-Manipulators dargestellt, wobei die Längsachse des Uterus-Manipulators bzw. die Längsachsen seiner Abschnitte parallel zur Zeichenebene liegen. Im Unterschied zu Figur 2 zeigt Figur 3 teilweise einen Querschnitt.

Der distale Abschnitt 13 umfasst einen Endballon 31 und einen Stützballon 32, die voneinander beabstandet angeordnet und jeweils im expandierten Zustand dargestellt sind. Abweichend von der Darstellung in den Figuren 2 und 3 können der Endballon 31 und der Stützballon 32 unmittelbar aneinander grenzen oder einen gemeinsamen Innenraum mit einer Taille zwischen Endballon 31 und Stützballon 32 aufweisen. Der Endballon 31 und der Stützballon 32 weisen jeweils eine Wand bzw. Haut aus Silicon, Gummi, einem anderen Elastomer oder einem beliebigen anderen, insbesondere biokompatiblen, elastischen Material auf. Der Endballon 31 umschließt ein distales Ende 55 eines nachfolgend mit Bezug auf Figur 3 näher beschriebenen Schaftabschnitts vollständig.

Im Endballon 31, im Stützballon 32 und zwischen dem Endballon 31 und dem Stützballon 32 ist ein insbesondere gerader Schaftabschnitt 33 mit einem insbesondere kreisförmigen oder elliptischen Querschnitt und einer Öffnung 34 angeordnet. Durch die Öffnung 34 kann beispielsweise ein Fluid zur Pertubation oder ein endoskopisches Instrument aus dem Schaftabschnitt 33 nach außen geschoben werden.

Ferner umfasst der distale Abschnitt 13 eine Kupplung 39 zur mechanischen (beispielsweise form- oder kraftschlüssigen) und funktionalen Kopplung des distalen Abschnitts 13 mit einem proximalen Abschnitt eines Uterus-Manipulators oder eines anderen mikroinvasiven medizinischen Instruments. Der hier mit Bezug auf die Figuren 2 und 3 dargestellte distale Abschnitt 13 unterscheidet sich von dem oben anhand der Figur 1 dargestellten durch die Kupplung 39, die anstelle des Gelenks 16 oder zusätzlich zu diesem vorgesehen sein kann. Ein Gelenk kann beispielsweise abweichend von den Figuren 2 und 3 zwischen der Kupplung 39 und dem Stützballon 32 angeordnet sein. Alternativ ist ein Gelenk beispielsweise proximal der Kupplung 39 in einem proximalen Abschnitt des Uterus-Manipulators angeordnet.

Der Endballon 31 und der Stützballon 32 sind mit unterbrochenen Linien und als zwei separate Bauteile dargestellt. Die elastischen Bauteile, die den Endballon 31 und den Stützballon 32 binden, sind beispielsweise formschlüssig, kraftschlüssig oder stoffschlüssig mit dem Schaftabschnitt 33 verbunden. Eine formschlüssige Verbindung umfasst beispielsweise einen oder mehrere Stege am Endballon 31 bzw. m Stützballon 32, der bzw. die in entsprechende Nuten am Schaftabschnitt 33 eingreifen. Eine kraftschlüssige Verbindung wird beispielsweise durch eine elastische Rückstellkraft des den Endballon 31 bzw. den Stützballon 32 bildenden Bauteils und Haftreibung zwischen diesem und dem Schaftabschnitt 33 erzeugt. Abweichend von der Darstellung in Figur 3 können der Endballon 31 und der Stützballon 32 von einem einzigen gemeinsamen Bauteil gebildet werden, in dem dann auch die Öffnung 34 angeordnet ist.

Ein Bereich 51 des Schaftabschnitts 33 ist in dem Endballon 31 angeordnet. Der Bereich 51 des Schaftabschnitts 33 umfasst das distale Ende des Schaftabschnitts 33, das von dem Endballon 31 vollständig umschlossen ist. Ein Bereich 52 des Schaftabschnitts 33 ist im Stützballon 32 angeordnet bzw. von diesem umgeben.

Im Schaftabschnitt 33 sind ein erster Kanal 61, ein zweiter Kanal 62 und ein dritter Kanal 64 angeordnet. Proximal enden die drei Kanäle 61, 62, 64 an der Kupplung 39. Die Kupplung 39 ist ausgebildet, um bei Kopplung des distalen Abschnitts 13 mit einem proximalen Abschnitt eines mikroinvasiven medizinischen Instruments die Lumina der Kanäle 61, 62, 64 mit entsprechenden Lumina korrespondierender Kanäle des proximalen Abschnitts zu verbinden.

Der erste Kanal 61 ist über eine Öffnung an seinem distalen Ende im Bereich 51 des Schaftabschnitts 33 mit dem Inneren bzw. dem Hohlraum des Endballons 31 verbunden. Der zweite Kanal 62 ist über eine Öffnung an seinem distalen Ende im Bereich 52 des Schaftabschnitts 33 mit dem Inneren bzw. dem Hohlraum des Stützballons 32 verbunden. Der dritte Kanal 64 ist mit der Öffnung 34 verbunden. Der erste Kanal 61 und der zweite Kanal 62 sind ausgebildet, um Fluide zu dem Endballon 31 bzw. zu dem Stützballon 32 oder von dem Endballon 31 und dem Stützballon 32 wegzuleiten. Anstelle zweier separater Kanäle 61, 62 kann ein gemeinsamer Kanal vorgesehen sein, der mit den Innenräumen des Endballons 31 und des Stützballons 32 verbunden ist. Der dritte Kanal 64 ist zum Leiten eines Fluids oder zur Führung eines Instruments zu der Öffnung 34 ausgebildet.

Das distale Ende 55 des Bereichs 51 des Schaftabschnitts 33 ist in Figur 3 mit einer halbkugelförmigen Gestalt dargestellt. Dabei weist die Oberfläche des distalen Endes 55 an jeder Stelle einen Krümmungsradius auf, der dem halben Durchmesser des Bereichs 51 des Schaftabschnitts 33 entspricht. Abweichend davon kann das distale Ende 55 andere Formen aufweisen. Zur Minimierung des Verletzungsrisikos weist das distale Ende 55 vorzugsweise eine Gestalt auf, bei der zumindest an allen distal orientierten Oberflächenbereichen der Krümmungsradius mindestens ein Viertel oder mindestens ein Drittel des Durchmessers des Bereichs 51 des Schaftabschnitts 33 aufweist. Im Fall eines nichtkreisförmigen Querschnitts des Bereichs 51 des Schaftabschnitts 33 beträgt der Krümmungsradius am distalen Ende 55 insbesondere mindestens ein Viertel oder mindestens ein Drittel der maximalen linearen Abmessung des Querschnitts des Bereichs 51 des Schaftabschnitts 33.

### Bezugszeichen

- 10: Uterus-Manipulator
- 12: proximaler Abschnitt des Uterus-Manipulators 10
- 13: distaler Abschnitt des Uterus-Manipulators 10
- 15: Schaft des Uterus-Manipulators 10
- 16: Gelenk im Schaft 15
- 20: Griff-Einrichtung
- 31: Endballon
- 32: Stützballon
- 33: Abschnitt des Schafts 15 zwischen Endballon und Stützballon
- 34: Öffnung am Abschnitt 33 des Schafts 15
- 39: Kupplung
- 51: Bereich des Schafts 15 im Endballon 31
- 52: Bereich des Schafts 15 im Stützballon 32
- 55: distales Ende des Bereichs 51
- 61: Kanal zum Endballon 31
- 62: Kanal zum Stützballon 32
- 64: Kanal zur Öffnung 34

## Patentansprüche

1. **Uterus-Manipulator** (10), mit:
einem **Schaft** (15) bestehend aus einem distalen und einem proximalen Schaftabschnitt, einem **Endballon** (31) an einem distalen Ende (55) des distalen Schaftabschnitts, wobei der Endballon (31) das distale Ende (55) des distalen Schaftabschnitts **vollständig umschließt,**
ferner mit einem **Stützballon** (32) am distalen Schaftabschnitt, der vom distalen Ende (55) des distalen Schaftabschnitts beabstandet ist,
ferner mit einer seitlichen **Öffnung** (34) am distalen Schaftabschnitt zwischen dem Endballon (31) und dem Stützballon (32), durch die ein Fluid geleitet oder ein Gegenstand aus dem distalen Schaftabschnitt nach außen geschoben werden kann,
ferner mit einem **ersten Kanal** (61) im oder am Schaft (15), dessen Lumen am distalen Ende mit dem Inneren des Endballons (31) verbunden ist;
einem **zweiten Kanal** (62) im oder am Schaft (15),
**dadurch gekennzeichnet, dass**
das Lumen des zweiten Kanals (62) am distalen Ende mit dem Inneren des Stützballons (32) verbunden ist
ferner mit einer **Kupplung** (39) zwischen dem zur mehrmaligen Verwendung
vorgesehenen proximalen Schaftabschnitt und dem zur einmaligen Verwendung vorgesehenen distalen Schaftabschnitt, wobei die Kupplung (39) zur mehrmaligen lösbaren Kopplung des proximalen Schaftabschnitts und des distalen Schaftabschnitts ausgebildet ist.

2. **Uterus-Manipulator** (10) nach einem der vorangehenden Ansprüche, ferner mit:
einem **Gelenk** (16) zwischen einem proximalen Abschnitt und einem distalen Abschnitt (33) des Schafts (15), wobei der Endballon (31) und ggf. der Stützballon (32) am distalen Abschnitt (33) des Schafts (15) angeordnet sind.

3. **Uterus-Manipulator** (10) nach einem der vorangehenden Ansprüche, wobei alle **Krümmungsradien** am distalen Ende (55) des Schafts (15) mindestens ein Viertel des Durchmessers des Schafts (15) betragen.

4. **Distaler Abschnitt** (13) für ein mikroinvasives medizinisches Instrument (10), mit:
einem **Schaftabschnitt** (33);
einem **Endballon** (31) an einem distalen Ende (55) des Schaftabschnitts (33),
wobei der Endballon (31) das distale Ende (55) des Schaftabschnitts (33) **vollständig umschließt,**
ferner miteinem **Stützballon** (32) am Schaftabschnitt (33), der vom distalen Ende (55) des Schaftabschnitts (33) beabstandet ist,
ferner mit:
einer seitlichen **Öffnung** (34) am Schaftabschnitt (33) zwischen dem Endballon (31) und dem Stützballon (32), durch die ein Fluid geleitet oder ein Gegenstand aus dem Schaftabschnitt (33) nach außen geschoben werden kann,
ferner mit:
einem **ersten Kanal** (61) im oder am Schaftabschnitt (33), dessen Lumen am distalen Ende mit dem Inneren des Endballons (31) verbunden ist;
einem **zweiten Kanal** (62) im oder am Schaftabschnitt (33),
**dadurch gekennzeichnet, dass**
das Lumen des zweiten Kanals (62) am distalen Ende mit dem Inneren des Stützballons (32) verbunden ist,
wobei der distale Abschnitt ferner eine **Kupplung** (39) zum Koppeln des distalen Abschnitts (13) mit einem proximalen Schaftabschnitt umfasst,
wobei der distale Abschnitt (13) für eine einmalige Verwendung und anschließende Entsorgung ausgebildet ist.

5. Distaler Abschnitt (13) nach Anspruch 4, wobei alle **Krümmungsradien** am distalen Ende (55) des Schaftabschnitts (33) mindestens ein Viertel des Durchmessers des Schaftabschnitts (33) betragen.

## Claims

1. Uterus manipulator (10) with:
a shaft (15) having a distal shaft portion and a proximal shaft portion,
an end balloon (31) at a distal end (55) of the distal shaft portion,
wherein the end balloon (31) completely encloses the distal end (55) of the distal shaft portion,
moreover with a support balloon (32) on the distal shaft portion, which support balloon (32) is spaced apart from the distal end (55) of the distal shaft portion,
moreover with a lateral opening (34) on the distal shaft portion between the end balloon (31) and the support balloon (32), through which lateral opening (34) a fluid can be conveyed or an item can be pushed out from the distal shaft portion,
moreover with a first channel (61) in or on the shaft (15), the lumen of which first channel (61) is connected at the distal end to the interior of the end balloon (31);
a second channel (62) in or on the shaft (15),
**characterized in that**
the lumen of the second channel (62) is connected at the distal end to the interior of the support balloon (32),
moreover with a coupling (39) between the proximal shaft portion, which is provided for repeated use, and the distal shaft portion, which is provided for a one-off use,
wherein the coupling (39) is designed to couple the proximal shaft portion and the distal shaft portion repeatedly in a releasable manner.

2. Uterus manipulator (10) according to one of the preceding claims, moreover with:
a joint (16) between a proximal portion and a distal portion (33) of the shaft (15), wherein the end balloon (31) and if appropriate the support balloon (32) are arranged on the distal portion (33) of the shaft (15).

3. Uterus manipulator (10) according to one of the preceding claims, wherein all the radii of curvature at the distal end (55) of the shaft (15) amount to at least one quarter of the diameter of the shaft (15).

4. Distal portion (13) for a microinvasive medical instrument (10), with:
a shaft portion (33);
an end balloon (31) at a distal end (55) of the shaft portion (33),
wherein the end balloon (31) completely encloses the distal end (55) of the shaft portion (33),
moreover with a support balloon (32) on the shaft portion (33), which support balloon (32) is spaced apart from the distal end (55) of the shaft portion (33),
moreover with:
a lateral opening (34) on the shaft portion (33) between the end balloon (31) and the support balloon (32), through which lateral opening (34) a fluid can be conveyed or an item can be pushed out from the shaft portion (33),
moreover with:
a first channel (61) in or on the shaft portion (33), the lumen of which first channel (61) is connected at the distal end to the interior of the end balloon (31);
a second channel (62) in or on the shaft portion (33),
**characterized in that**
the lumen of the second channel (62) is connected at the distal end to the interior of the support balloon (32),
wherein the distal portion moreover comprises a coupling (39) for coupling the distal portion (13) to a proximal shaft portion,
wherein the distal portion (13) is designed for a one-off use and subsequent disposal.

5. Distal portion (13) according to Claim 4, wherein all the radii of curvature at the distal end (55) of the shaft portion (33) amount to at least one quarter of the diameter of the shaft portion (33).

## Revendications

1. Manipulateur d'utérus (10), comprenant :
une tige (15) constituée d'une portion de tige distale et d'une portion de tige proximale,
un ballonnet d'extrémité (31) à une extrémité distale (55) de la portion de tige distale,
le ballonnet d'extrémité (31) entourant complètement l'extrémité distale (55) de la portion de tige distale,
comprenant en outre un ballonnet de support (32) au niveau de la portion de tige distale, qui est espacé de l'extrémité distale (55) de la portion de tige distale,
comprenant en outre une ouverture latérale (34) au niveau de la portion de tige distale entre le ballonnet d'extrémité (31) et le ballonnet de support (32), à travers laquelle un fluide peut être conduit ou un objet peut être poussé vers l'extérieur depuis la portion de tige distale,
comprenant en outre un premier canal (61) dans ou sur la tige (15), dont la lumière à l'extrémité distale est connectée à l'intérieur du ballon d'extrémité (31) ;
un deuxième canal (62) dans ou sur la tige (15),
**caractérisé en ce que**
la lumière du deuxième canal (62) à l'extrémité distale est connectée à l'intérieur du ballon de support (32),
un accouplement (39) étant en outre prévu entre la portion de tige proximale prévue pour une utilisation multiple et la portion de tige distale prévue pour une utilisation unique,
l'accouplement (39) étant réalisé de manière à permettre un accouplement desserrable plusieurs fois de la portion de tige proximale et de la portion de tige distale.

2. Manipulateur d'utérus (10) selon l'une quelconque des revendications précédentes, comprenant en outre :
une articulation (16) entre une portion proximale et une portion distale (33) de la tige (15), le ballonnet d'extrémité (31) et éventuellement le ballonnet de support (32) étant disposés au niveau de la portion distale (33) de la tige (15).

3. Manipulateur d'utérus (10) selon l'une quelconque des revendications précédentes, dans lequel tous les rayons de courbure à l'extrémité distale (55) de la tige (15) valent au moins un quart du diamètre de la tige (15).

4. Portion distale (13) pour un instrument médical micro-invasif (10), comprenant :
une portion de tige (33) ;
un ballonnet d'extrémité (31) à une extrémité distale (55) de la portion de tige (33),
le ballonnet d'extrémité (31) entourant complètement l'extrémité distale (55) de la portion de tige (33),
comprenant en outre un ballonnet de support (32) au niveau de la portion de tige (33), lequel est espacé de l'extrémité distale (55) de la portion de tige (33),
comprenant en outre :
une ouverture latérale (34) au niveau de la portion de tige (33) entre le ballonnet d'extrémité (31) et le ballonnet de support (32),
à travers laquelle un fluide est guidé ou un objet peut être poussé vers l'extérieur depuis la portion de tige (33),
comprenant en outre :
un premier canal (61) dans ou sur la portion de tige (33), dont la lumière à l'extrémité distale est connectée à l'intérieur du ballonnet d'extrémité (31) ;
un deuxième canal (62) dans ou sur la portion de tige (33),
**caractérisée en ce que**
la lumière du deuxième canal (62) à l'extrémité distale est connectée à l'intérieur du ballonnet de support (32),
la portion distale comprenant en outre un accouplement (39) pour l'accouplement de la portion distale (13) à une portion de tige proximale,
la portion distale (13) étant réalisée pour une utilisation unique et une mise au rebut subséquente.

5. Portion distale (13) selon la revendication 4, dans laquelle tous les rayons de courbure à l'extrémité distale (55) de la portion de tige (33) valent au moins un quart du diamètre de la portion de tige (33).
